(19) Europäisches Patentamt
European Patent Office
Office européen des brevets

(11) EP 4 161 557 B1

(12) **EUROPEAN PATENT SPECIFICATION**

(45) Date of publication and mention of the grant of the patent:
**11.03.2026 Bulletin 2026/11**

(21) Application number: **20918125.4**

(22) Date of filing: **04.06.2020**

(51) International Patent Classification (IPC):
***A61K 38/13*** (2006.01) ***A61P 31/14*** (2006.01)
***A61K 45/06*** (2006.01) ***C07K 7/64*** (2006.01)

(52) Cooperative Patent Classification (CPC):
(C-Sets available)
**A61P 31/14; A61K 38/13; A61K 45/06; C07K 7/645**
(Cont.)

(86) International application number:
**PCT/CN2020/094431**

(87) International publication number:
**WO 2021/243658 (09.12.2021 Gazette 2021/49)**

# (54) WS-635 FOR USE IN THE TREATMENT OR PREVENTION OF SARS-COV-2 INFECTION

WS-635 ZUR VERWENDUNG IN DER BEHANDLUNG ODER PRÄVENTION EINER SARS-COV-2 INFEKTION

WS-635 POUR L'UTILISATION DANS LE TRAITEMENT OU PRÉVENTION D'UNE INFECTION PAR SARS-COV-2

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.04.2023 Bulletin 2023/15**

(73) Proprietor: **Waterstone Pharmaceuticals (Wuhan) Co., Ltd.**
**Wuhan, Hubei 430075 (CN)**

(72) Inventors:
- **CUI, Jian**
  **Wuhan, Hubei 430075 (CN)**
- **HU, Minglong**
  **Wuhan, Hubei 430075 (CN)**
- **YU, Yao**
  **Wuhan, Hubei 430075 (CN)**
- **ZHAO, Along**
  **Wuhan, Hubei 430075 (CN)**
- **ZHANG, Faming**
  **Wuhan, Hubei 430075 (CN)**

(74) Representative: **Maiwald GmbH**
**Elisenhof**
**Elisenstraße 3**
**80335 München (DE)**

(56) References cited:
**WO-A1-2012/075494** **WO-A1-2015/161908**
**WO-A1-2017/156262** **WO-A1-2017/200984**
**WO-A1-2020/037530** **WO-A1-2020/038426**
**WO-A2-2014/145686** **CN-A- 112 153 978**

- **JULIE DYALL ET AL: "Middle East Respiratory Syndrome and Severe Acute Respiratory Syndrome: Current Therapeutic Options and Potential Targets for Novel Therapies", DRUGS, vol. 77, no. 18, 15 November 2017 (2017-11-15), NZ, pages 1935 - 1966, XP055731800, ISSN: 0012-6667, DOI: 10.1007/s40265-017-0830-1**
- **COUR MARTIN, OVIZE MICHEL, ARGAUD LAURENT: "Cyclosporine A: a valid candidate to treat COVID-19 patients with acute respiratory failure?", CRITICAL CARE, vol. 24, no. 1, 1 December 2020 (2020-12-01), XP055878377, DOI: 10.1186/s13054-020-03014-1**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 161 557 B1

(52) Cooperative Patent Classification (CPC): (Cont.)

C-Sets
**A61K 38/13, A61K 2300/00**

## Description

## FIELD OF THE INVENTION

The present invention relates to the field of medicinal technology, in particular, to the treatment or prevention of Coronaviridae infection in a subject, wherein the Coronaviridae is 2019-nCov virus.

## BACKGROUND OF THE INVENTION

The World Health Organization has recently declared the severe acute respiratory syndrome coronavirus 2 (SARS-Cov-2) a public health emergency of international concern. As of June 1, 2020, there are more than 6,000,000 confirmed cases and more than 370,000 death cases all around the world.

Coronaviridae (also may be known as "Coronaviruses") is a family of enveloped, positive-sense, single-stranded RNA viruses. Coronaviruses may cause diseases in mammals and birds. In humans, the viruses cause respiratory infections, including the common cold, which are typically mild, though rarer forms such as SARS (including the one causing COVID-19) and MERS can be lethal.

Therefore there is an increasing demand for the research and development of more effective method for treating or preventing a Coronaviridae infection.

## SUMMARY OF THE INVENTION

The following is only an overview of some aspects of the present invention, but is not limited thereto. The references to the methods of treatment by therapy in this description are to be interpreted as references to compounds, pharmaceutical compositions and medicaments of the present invention for use in those methods.

In the research and development process, the inventors surprisingly found that WS-635 (the compound of formula (I)) showed significantly high efficiency and activity for treating or preventing a Coronaviridae infection in several experimental tests.

In one aspect of present disclosure, there is provided a method of treating or preventing a Coronaviridae infection in a subject comprising administrating a therapeutically effective amount of a compound of Formula I or a stereoisomer, a tautomer, an N-oxide, a solvate or a pharmaceutically acceptable salt thereof to the subject in need thereof,

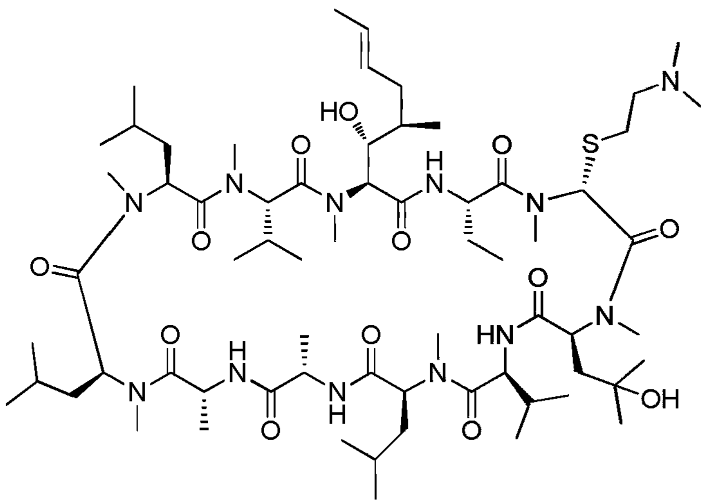

(I).

and the Coronaviridae is 2019-nCov virus.

In another aspect of present disclosure, there is provided a pharmaceutical composition for treating or preventing Coronaviridae virus infection comprising a compound of Formula I or a stereoisomer, a tautomer, an N-oxide, a solvate or a pharmaceutically acceptable salt thereof,

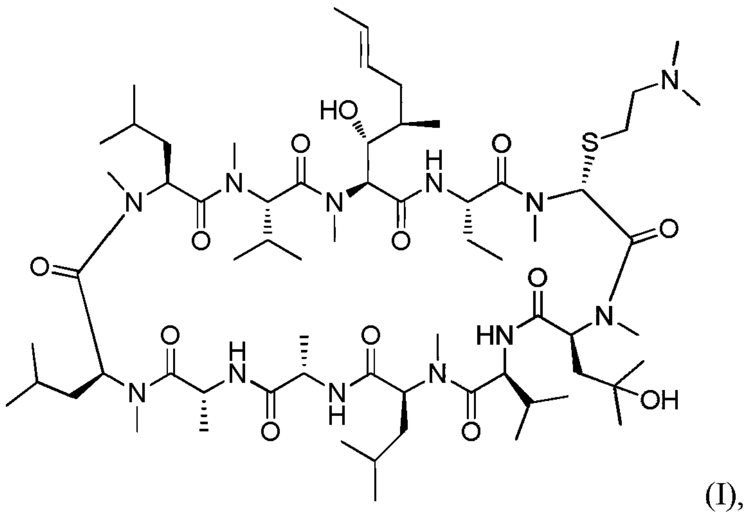

(I),

and the Coronaviridae is 2019-nCov virus.

In another aspect of present disclosure, there is provided compound of Formula I or a stereoisomer, a tautomer, an N-oxide, a solvate or a pharmaceutically acceptable salt thereof, for use in treating or preventing Coronaviridae infection in a subject

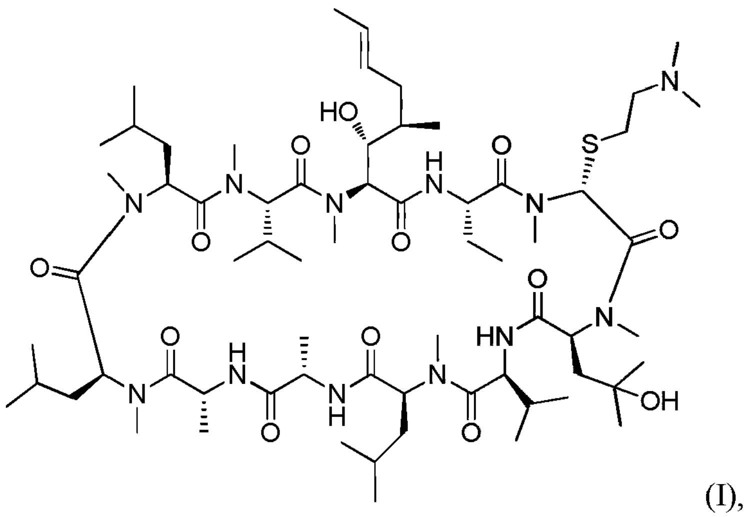

(I),

the Coronaviridae is 2019-nCov virus.

The foregoing merely summarizes certain aspects disclosed herein and is not intended to be limiting in nature. These aspects and other aspects and additional embodiments, features, and advantages of the invention will be apparent from the following detailed description and through practice of the invention.

## BRIEF DESCRIPTION OF THE DRAWINGS

These and other aspects and advantages of embodiments of the present disclosure will become apparent and more readily appreciated from the following descriptions made with reference the accompanying schemes and drawings, in which:

Fig.1 shows the inhibitory effect of WS-635 on the SARS-CoV-2 coronavirus at the cellular level;
Fig.2 shows WS-635 anti SARS-CoV-2 activity results; and
Fig.3 shows WS-635 anti human coronavirus (HCoV) 229E activity results.

## EMBODIMENTS

**Definition**

Unless otherwise indicated, otherwise the following terms and phrases as used herein will have the following meanings:

"Esters" refers to any ester of the compound, wherein the molecule of any-COOH functional group is-C(O) OR function with a substituent, or wherein any of the molecule-OH functional group is-OC(O) R functional groups, wherein R may be formed a stable ester moiety of the ester moiety is any carbon-containing groups, including but not limited to alkyl, alkenyl, alkynyl, cycloalkyl, cycloalkylalkyl, aryl, arylalkyl, heterocyclyl, heterocycloalkyl and substituted derivatives thereof.

"Pharmaceutically acceptable" refers to a suitable for use in a pharmaceutical formulation, generally regarded as safe, a regulatory agency of the country or a state government official authorized to be used by, or column in the Pharmacopeia or other generally recognized pharmacopeia for use in animals and particularly for use in humans.

"Pharmaceutically acceptable carrier" refers to a pharmaceutically acceptable diluent, adjuvant, excipient or carrier or other component, and is administered in combination with a compound of the present invention.

"Pharmaceutically acceptable salt" refers to the desired pharmacologically active salt thereof can be enhanced. Pharmaceutically acceptable salts thereof with inorganic or organic acids include the acid addition salts, metal salts and amine salts. Acid addition salts may be formed with inorganic acids and organic acids.

"A therapeutically effective amount" means the compound was administered to an animal for the treatment of diseases, the treatment of the disease in an amount sufficient to effect.

A therapeutically effective amount of a compound "treating" means and includes any of the application:

(1) prevention may be predisposed to the disease but still does not yet experience or display the pathology or symptomatology of the disease in animals that develop the disease,

(2) inhibiting the disease is experiencing or displaying the pathology or symptomatology of the disease in an animal of the (i.e., arresting further development of the pathology and/or symptomatology), or

(3) improve the pathology or symptomatology of the disease is experiencing or displaying the disease in an animal (i.e., reversing the pathology and/or symptomatology).

**COMPOUND**

The present disclosure relates to a compound of Formula I or a stereoisomer, a tautomer, an N-oxide, a solvate or a pharmaceutically acceptable salt thereof,

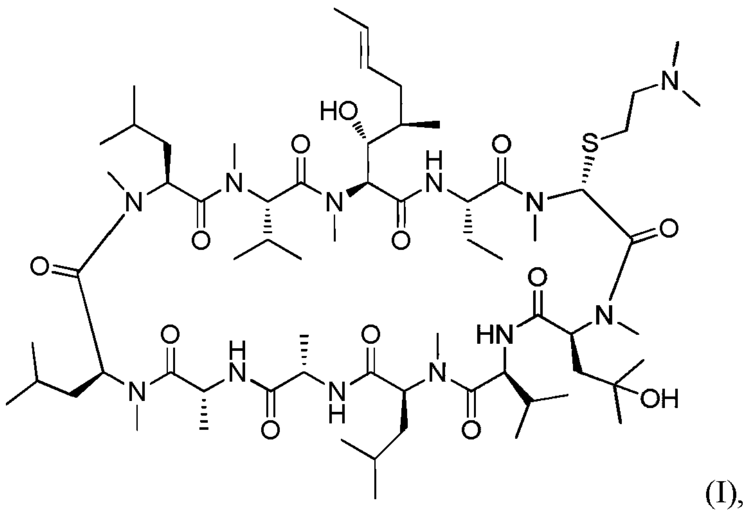

(I),

a stereoisomer, a tautomer, an N-oxide, a solvate or a pharmaceutically acceptable salt when treating or preventing a Coronaviridae wherein Coronaviridae is 2019-nCov virus.

In some examples of present disclosure, the compound of Formula I may be used in a form a pharmaceutically acceptable salt, and the pharmaceutically acceptable salt is at least one selected from a group comprising phosphate, hydrochloride, hydrobromide, hydroiodide, sulfate, nitrate, ethanesulfonate, toluenesulfonate and benzenesulfonate, acetate, trifluoroacetate, tartrate, maleate, succinate, citrate, benzoate, salicylate and ascorbate, adipate, alginate,

arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate, galacturonate, glucoheptaoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate.

**Pharmaceutical Formulation and Use**

The compound of present disclosure may be formulated with conventional carriers and excipients, carriers and excipients which will meet the selected common practice. Tablets contain excipients, glidants, fillers, binders and the like. An aqueous formulation will be made in the form of a sterile, when it is intended for administration of the administered orally, typically isotonic solution.

While the active ingredient can be administered alone, but may preferably be formulated into pharmaceutical formulation. The formulations of the present invention, whether for human or veterinary use in formulations, each having at least one active ingredient, and one or more acceptable carriers and optionally other therapeutic ingredients.

The present invention suitable for oral administration of the formulations may be presented as discrete units, each containing a predetermined quantity of active ingredient such as capsules, cachets or tablets; a powder or granules; in an aqueous or non-aqueous liquid solutions or suspensions; or oil in water emulsion or a water in oil emulsion. The active ingredients may also be a bolus, electuary or paste form.

An effective amount of an active ingredient at least depending upon the nature of the disease being treated, toxicity, the compound is used for prophylaxis (low dose) or for the active viral infection, pharmaceutical formulation and administration method, and then using a conventional dose escalation studies by the clinician to make a decision.

In some preferred embodiments of present disclosure, the present disclosure relates to the compound of Formula I. It is surprisingly found by the inventors that the compound of Formula I showed significantly high efficiency and activity for treating or preventing a Coronaviridae infection in several experimental tests.

Then the compound of Formula I or the pharmaceutically acceptable salt, ester or prodrug thereof may be administrated to the subject in a form of sol, aromatic water, aerosol, partial inhalant, powder, granule, tablets, ointment, paste, emulsion, suspension, gas dispersion, solid dispersion, microparticle, pill. And in some examples, the compound of Formula I or a pharmaceutically acceptable salt, ester or prodrug thereof may be administrated at a daily dose of lower than 1000 mg, for example 100 mg to 1000 mg, 100~500 mg in term of the compound of Formula I.

Additionally the person skilled in the art may understand that combination therapy may also be used. The compounds of the compound of Formula I or a pharmaceutically acceptable salt or ester thereof may be in combination with one or more active agents. Suitable active agents for use in combination a non-limiting embodiments include one or more selected from the group comprising a corticosteroid, an anti-inflammatory signal transduction modulator, a β2-adrenoreceptor agonist bronchodilator, an anticholinergic, a mucolytic agent, hypertonic saline, agent for reducing cytokine storm, other drugs for treating a covid-19 virus infection, or mixtures thereof. Then there is is provided a method of treating or preventing covid-19 virus infection in a subject comprising administrating a therapeutically effective amount of a compound of Formula I or a pharmaceutically acceptable salt, ester or hydrate thereof to the subject in need thereof

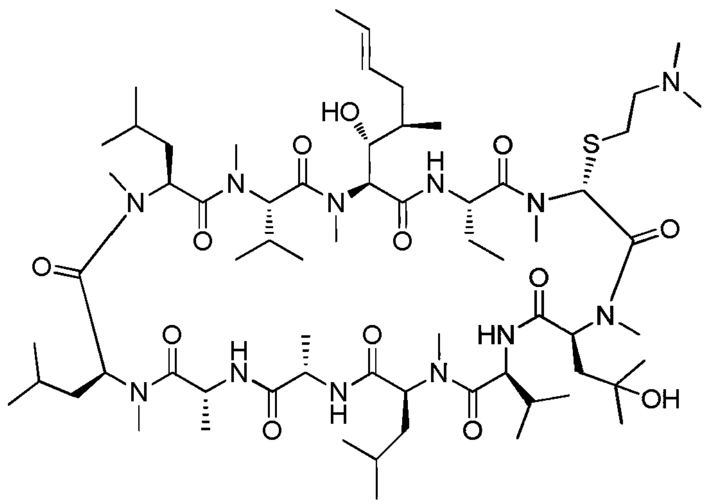

(I),

And in some specific examples, it is provided that the method may further comprise administering a therapeutically effective amount of at least one other therapeutic agent or composition thereof selected from the group comprising a corticosteroid, an anti-inflammatory signal transduction modulator, a β2-adrenoreceptor agonist bronchodilator, an anticholinergic, a mucolytic agent, hypertonic saline, agent for reducing cytokine storm, other drugs for treating a covid-19 virus infection, or mixtures thereof.

And in some specific examples, the compound of Formula I or the pharmaceutically acceptable salt, ester or hydrate thereof is administrated to the subject in a form of sol, aromatic water, aerosol, partial inhalant, powder, granule, tablets, ointment, paste, emulsion, suspension, gas dispersion, solid dispersion, microparticle, pill.

And in some specific examples, compound of Formula I or a pharmaceutically acceptable salt, ester or hydrate thereof is administrated at a daily dose of lower than 1000 mg in term of the compound of Formula I.

And in some specific examples, the compound of Formula I or a pharmaceutically acceptable salt, ester or hydrate thereof is administrated at a daily dose of 100 mg to 1000 mg in term of the compound of Formula I.

And in some specific examples, the compound of Formula I or a pharmaceutically acceptable salt, ester or hydrate thereof is administrated at a daily dose of 100 mg to 500 mg in term of the compound of Formula I.

In some other examples, it is provided a pharmaceutical composition to treat or prevent covid-19 virus infection comprising a compound of Formula I or a pharmaceutically acceptable salt, ester or hydrate thereof to the subject in need thereof

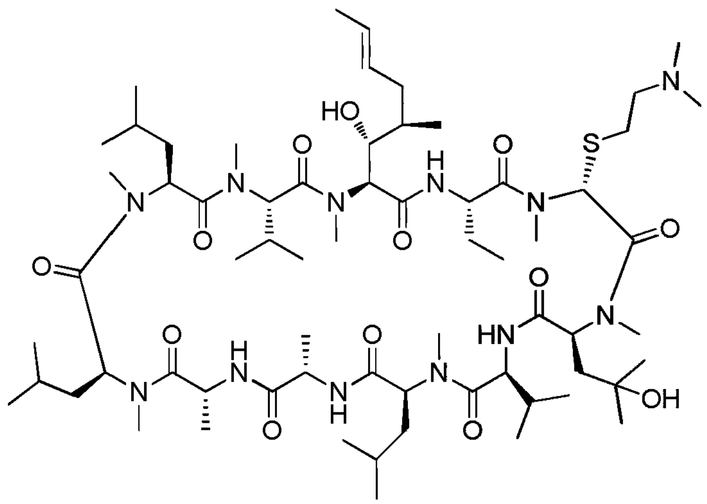

(I),

And in some specific examples, the composition further comprises at least one other therapeutic agent or composition thereof selected from the group comprising a corticosteroid, an anti-inflammatory signal transduction modulator, a β2-adrenoreceptor agonist bronchodilator, an anticholinergic, a mucolytic agent, hypertonic saline, agent for reducing cytokine storm, other drugs for treating a covid-19 virus infection, or mixtures thereof.

And in some specific examples, the pharmaceutically acceptable salt is at least one selected from a group comprising phosphate, hydrochloride, hydrobromide, hydroiodide, sulfate, nitrate, ethanesulfonate, toluenesulfonate and benzenesulfonate, acetate, trifluoroacetate, tartrate, maleate, succinate, citrate, benzoate, salicylate and ascorbate, adipate, alginate, arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate, galacturonate, glucoheptaoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate.

And in some specific examples, the compound of Formula I or the pharmaceutically acceptable salt, ester or hydrate thereof is in a form of sol, aromatic water, aerosol, partial inhalant, powder, granule, tablets, ointment, paste, emulsion, suspension, gas dispersion, solid dispersion, microparticle, pill.

And in some specific examples, the composition comprises the compound of Formula I or the pharmaceutically acceptable salt, ester or hydrate thereof at a dose of lower than 1000 mg, lower than 800 mg, lower than 600 mg, lower than 500 mg, or lower than 200 mg.

Then it is provided in the present disclosure compound of Formula I or a pharmaceutically acceptable salt, ester or hydrate thereof, for use in treating or preventing covid-19 virus infection in a subject,

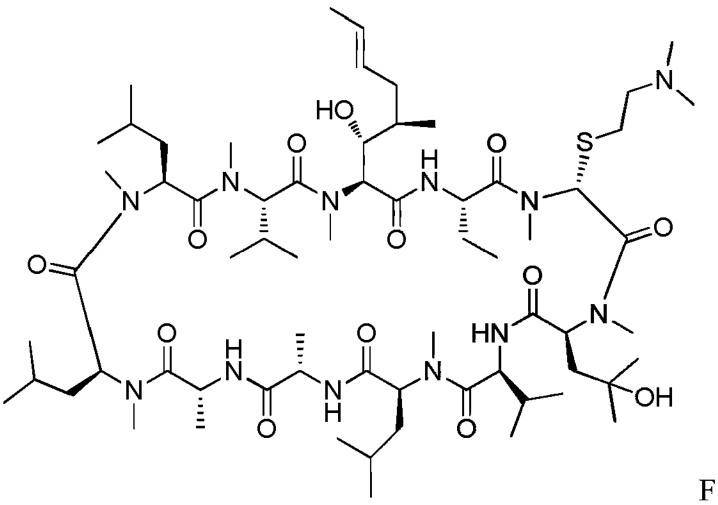

Formula (I),

And in some specific examples, the pharmaceutically acceptable salt is at least one selected from a group comprising phosphate, hydrochloride, hydrobromide, hydroiodide, sulfate, nitrate, ethanesulfonate, toluenesulfonate and benzenesulfonate, acetate, trifluoroacetate, tartrate, maleate, succinate, citrate, benzoate, salicylate and ascorbate, adipate, alginate, arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate, galacturonate, glucoheptaoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate.

And in some specific examples, the compound of Formula I or the pharmaceutically acceptable salt, ester or hydrate thereof is administrated to the subject in a form of sol, aromatic water, aerosol, partial inhalant, powder, granule, tablets, ointment, paste, emulsion, suspension, gas dispersion, solid dispersion, microparticle, pill.

And in some specific examples, the compound of Formula I or a pharmaceutically acceptable salt, ester or hydrate thereof is administrated at a daily dose of lower than 1000 mg in term of the compound of Formula I.

And in some specific examples, the compound of Formula I or a pharmaceutically acceptable salt, ester or hydrate thereof is administrated at a daily dose of 100 mg to 1000 mg in term of the compound of Formula I.

And in some specific examples, the compound of Formula I or a pharmaceutically acceptable salt, ester or hydrate thereof is administrated at a daily dose of 100 mg to 500 mg in term of the compound of Formula I.

## Examples

### Example 1. Evaluating the inhibitory effect of WS-635 on the SARS-CoV-2 coronavirus at the cellular level.

WS-635 provided by Waterstone Pharmaceuticals Inc. as powder.

Virus cells are preserved by the Virology Laboratory of the Institute of Microbiology and Epidemiology, Academy of Military Medical Sciences.

SARS-CoV-2 coronavirus isolates (with deposition code: 2019-nCoV BetaCoV/Beijing/AMMS01/2020) are preserved by Institute of Microbiology and Epidemiology, Academy of Military Medical Sciences.

Determination of $EC_{50}$ of WS-635 by viral nucleic acid quantification:
Human Vero cell was purchased from ATCC (US), The SARS-Cov-2 virus was isolated by Beijing Military Medical Research Institute with the collection deposition code: 2019-nCoV BetaCoV/Beijing/AMMS01/2020.

1). The $CC_{50}$ of the drug was determined by the method of MTS (Promega). The details are as follows: Vero cells were inoculated into 96 well cell culture plate at 10000 / well concentration, and cultured overnight at 37 °C and 5% $CO_2$. When the cell grew to a full monolayer, 2% FBS DMEM maintenance solution containing different concentrations of the drug (initial concentration of 400 μM) was added to 100 μL / well, and 4 multiple pores were measured at each concentration. Continue to culture and observe the cell status under the microscope every day. Add 20 μL MTS solution until the 4th day of dosing, incubate at 37 °C for 1 hour, and measure the OD490 value.

2). $EC_{50}$ of the drug was determined by nucleic acid quantitative method. As follows: Vero cells were inoculated into 96 well plates at a concentration of 10000 / well one day in advance. Dilute the 2019-nCoV virus with 2% cell maintenance

medium and add it to a 96-well plate to make the virus content of each well is 100 $TCID_{50}$. WS-635 solubilized in DMSO was prepared into 400, 200, 100, 50, 25 and 12.5 μM with DMEM maintenance solution of 2% FBS. The cell culture supernatant was discarded, and T705 (100 μL / well) of different concentrations was added. Each drug had 4 multiple holes, and then 100 $TCID_{50}$ virus solution was added to each drop. The positive drug control (10 μM Remdesivir (RDV)), virus control and normal cell control groups were set up and cultured in 37 °C, 5% $CO_2$ incubator. On the second day after infection, 50 μL cell supernatant was taken from each pore to extract nucleic acid. The viral load was detected by quantitative RT-PCR and $EC_{50}$ was calculated by fitting the dose-response curve with graphpad prism 7. 3).

$$\text{Selective Index SI} = CC_{50} / EC_{50}.$$

Test the inhibitory effect of WS-635 on SARS-CoV-2 novel coronavirus at the cellular level by nucleic acid quantification method was used, and calculate the $EC_{50}$ of the WS-635.

The Figure 1 shows that WS-635 has an inhibitory effect on the 2019-nCoV new coronavirus at the cellular level, its $EC_{50}$ is 14.28 μM (Fig). $CC_{50}$ >100 μM, SI >7.

**Example 2. Ex vivo assay of WS-635 for SARS-CoV-2 replication**

Experiment was carried out by Chinese Institute of Virology at a P3 laboratory:
Human Vero Cells (ATCC) were infected with virus SARS-CoV-2 (2019-nCOV BetaCoV/Beijing/AMMS01/2020) to viral concentration of 100 $TCID_{50}$. Viral RNA load were measured with incubation at different compound concentrations.

Vero cells were inoculated into 96 well plates at a concentration of 10000 / well one day in advance. Dilute the 2019-nCoV virus with 2% cell maintenance medium and add it to a 96-well plate to make the virus content of each well is 100 $TCID_{50}$. WS-635 solubilized in DMSO was prepared into 400, 200, 100, 50, 25 and 12.5 μM with DMEM maintenance solution of 2% FBS. The cell culture supernatant was discarded, and T705 (100 μL / well) of different concentrations was added. Each drug had 4 multiple holes, and then 100 $TCID_{50}$ virus solution was added to each drop. The positive drug control (10 μM Remdesivir (RDV)), virus control and normal cell control groups were set up and cultured in 37 °C, 5% $CO_2$ incubator. On the second day after infection, 50 μL cell supernatant was taken from each pore to extract nucleic acid. The viral load was detected by quantitative RT-PCR and $EC_{50}$ was calculated by fitting the dose-response curve with graphpad prism 7.

Inhibition and cytotoxicity curves are shown in Figure 2 and Table 1. The figure shows that WS-635 inhibits SARS-CoV-2 (2019-nCOV BetaCoV/Beijing/AMMS01/2020) with a dose-dependent manner.

**Table 1:**

| CPD ID | $EC_{50}$ (μM) |
|---|---|
| WS-635 | 5.5 |

Reference example

**3. Evaluating the antiviral activity of test compound WS-635 against human coronavirus (HCoV) 229E in a cytopathic effect (CPE) assay.**

**3.1. Test and reference compounds**

Test compound WS-635 was provided by the sponsor in dry powders and prepared as 60 mM stock solutions in 100% DMSO solution. Reference compound remdesivir was provided by WuXi AppTec. Compounds were tested at 8 concentrations, 3-fold dilutions, in duplicate for 50% effective concentration ($EC_{50}$) and 50% cytotoxicity concentration ($CC_{50}$) determinations. The highest concentrations tested were listed in Table 2. The final concentration of DMSO in cell culture was 0.5%.

**Table 2. Information of the compounds**

| Compound ID | Weight (mg) | Purity (%) | MW | Aspect | The highest testing concentration for $EC_{50}$ | The highest testing concentration for $CC_{50}$ |
|---|---|---|---|---|---|---|
| WS-635 | 11.55 | 100 | 1321.8 | Powder | 100 μM | 300 μM |

(continued)

| Compound ID | Weight (mg) | Purity (%) | MW | Aspect | The highest testing concentration for $EC_{50}$ | The highest testing concentration for $CC_{50}$ |
|---|---|---|---|---|---|---|
| Remdesivir | 10.31 | 99 | 602.2 | Powder | 1 μM | 100 μM |

### 3.2. Cell line and Virus strain

HCoV 229E (ATCC VR-740) and MRC-5 cells (ATCC CCL-171) were acquired from the ATCC. MRC-5 cells are maintained in the Minimum Essential Medium (Sigma) supplemented with 10% FBS (Hyclone), 1% L-glutamine (Gibco), 1% NEAA (Gibco) and 1% penicillin-streptomycin (Hyclone). Minimum Essential Medium supplemented with 5% FBS, 1% L-glutamine, 1% NEAA and 1% penicillin-streptomycin was used as the assay medium.

### 3.3. Reagents and instruments

The main reagent used in this assay was luminescent cell viability assay kit CellTiter Glo (Promega). The main instrument used in this assay was Microplate Reader Synergy2 (BioTek).

### 3.4. Methods

The antiviral assay is summarized in Table 3.

**Table 3. Method of the antiviral assay**

| Virus | Cells | Treatment time (day)/Endpoint | Reference compound | Detection reagent |
|---|---|---|---|---|
| HCoV 229E | MRC-5 | 3/CPE | Remdesivir | Cell Titer Glo |

#### 3.4.1. HCoV 229E CPE assay

In 96-well plates, MRC-5 cells were seeded at 20,000 cells per well and cultured at 37°C and 5% $CO_2$ overnight. Next day, the medium containing serially diluted compounds and virus (200 $TCID_{50}$ per well) was added. The resulting cultures were kept at 35°C and 5% $CO_2$ for additional 3 days until that virus infection in the virus control (cells infected with virus, without compound treatment) displays significant CPE. Cell viability was measured with CellTiter Glo according to the manufacturer's manual. The luminescent signal was measured by Microplate Reader Synergy2 (Molecular Device). The antiviral activity of each compound was calculated based on the inhibition of CPE at each concentration normalized by the virus control.

#### 3.4.2. Cytotoxicity assay

Cytotoxicity of compounds was assessed under the same conditions but without virus infection, in parallel. Cell viability was measured with Cell Titer Glo according to the manufacturer's manual. $CC_{50}$ values were then calculated based on cytotoxicity at the test concentrations normalized by the medium control (medium only).

#### 3.4.3. Data analysis

Antiviral activity and cytotoxicity of compounds were expressed as % inhibition and % cell viability, respectively, and calculated with the formulas below:

$$Inhibition\ (\%) = (Raw\ data_{CPD} - Average_{VC}) / (Average_{CC} - Average_{VC}) \times 100$$

$$Cell\ Viability\ (\%) = (Raw\ data_{CPD} - Average_{MC}) / (Average_{CC} - Average_{MC}) \times 100$$

Where the Raw data $_{CPD}$ indicates the values of the compound-treatment wells; Average $_{VC}$, Average cc and Average $_{MC}$ indicate the average values of the virus control, cell control (cells without virus infection or compound treatment) and medium control, respectively.

$EC_{50}$ and $CC_{50}$ values were calculated using GraphPad Prism software (Version 5) and using the equation log(inhibitor) vs. response with variable slope. SI ($CC_{50}/EC_{50}$) was then calculated.

### 3.5. Results and Discussions

The antiviral experiment was quality controlled with reference compound. Remdesivir showed expected antiviral activity and effect on cell viability, indicating reliability of the experiment. The antiviral and cytotoxic results of test compounds are summarized in Table 3.

WS-635 showed inhibitory activity against HCoV 229E with an $EC_{50}$ value of 5.16 μM. It showed obvious cytotoxicity on MRC-5 cells with $CC_{50}$ and SI value of 46.79 μM and 9.07, respectively.

Inhibition and cytotoxicity curves are shown in Figure 3 and Table 4. The figure shows that WS-635 has obvious cytotoxicity on MRC-5 cells infected with HCoV 229E and works by blocking the spread of the virus.

**Table 4. $EC_{50}$ and $CC_{50}$ values of WS-635**

| CPD ID | $EC_{50}$ (μM) | $CC_{50}$ (μM) | SI ($CC_{50}/EC_{50}$) |
|---|---|---|---|
| WS-635 | 5.16 | 46.79 | 9.07 |
| Remdesivir | 0.03 | 49.25 | 1490.62 |

Reference throughout this specification to "an embodiment", "some embodiments", "one embodiment", "another example", "an example", "a specific example" or "some examples" means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples.

Although explanatory embodiments have been shown and described, it would be appreciated by those skilled in the art that the above embodiments cannot be construed to limit the present disclosure, and changes, alternatives, and modifications can be made in the embodiments without departing from principles and scope of the present disclosure.

## Claims

1. A compound of Formula I or a stereoisomer, a tautomer, an N-oxide, a solvate, or a pharmaceutically acceptable salt thereof for use in treating or preventing Coronaviridae infection in a subject, wherein a therapeutically effective amount of the compound of Formula I or a stereoisomer, a tautomer, an N-oxide, a solvate, or a pharmaceutically acceptable salt thereof is administered to the subject,

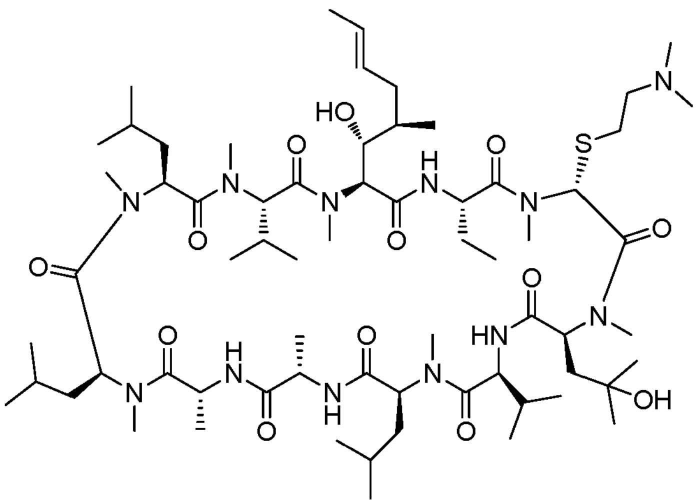

(I),

and the Coronaviridae is 2019-nCov virus.

2. The compound for use according to claim 1, wherein a therapeutically effective amount of at least one other therapeutic agent or composition thereof selected from the group comprising a corticosteroid, an anti-inflammatory signal transduction modulator, a β2-adrenoreceptor agonist bronchodilator, an anticholinergic, a mucolytic agent, hypertonic saline, agent for reducing cytokine storm, other drugs for treating a covid-19 virus infection, or mixtures thereof is also administered to the subject.

3. The compound for use according to claim 1 or 2, wherein the compound of Formula I or a stereoisomer, a tautomer, an N-oxide, a solvate, or a pharmaceutically acceptable salt thereof is administrated to the subject in a form of sol, aromatic water, aerosol, partial inhalant, powder, granule, tablets, ointment, paste, emulsion, suspension, gas dispersion, solid dispersion, micro particle, pill.

4. The compound for use according to any one of claims 1 to 3, wherein the pharmaceutically acceptable salt is at least one selected from a group comprising phosphate, hydrochloride, hydrobromide, hydroiodide, sulfate, nitrate, ethanesulfonate, toluenesulfonate and benzenesulfonate, acetate, trifluoroacetate, tartrate, maleate, succinate, citrate, benzoate, salicylate and ascorbate, adipate, alginate, arginate, aspartate, bisulfate, bisulfite, bromide, butyrate, camphorate, camphorsulfonate, caprylate, chloride, chlorobenzoate, cyclopentanepropionate, digluconate, dihydrogenphosphate, dinitrobenzoate, dodecylsulfate, fumarate, galacterate, galacturonate, glucoheptaoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate and phthalate.

5. The compound for use according to any one of claims 1 to 4, wherein the compound of Formula I or a stereoisomer, a tautomer, an N-oxide, a solvate, or a pharmaceutically acceptable salt thereof is administrated to the subject together with one or more pharmaceutically acceptable carriers, diluents or excipients.

6. The compound for use according to any one of claims 1 to 5, wherein the compound of Formula I or a pharmaceutically acceptable salt, or ester thereof is administrated at a daily dose of lower than 1000 mg in term of the compound of Formula I.

7. The compound for use according to any one of claims 1 to 6, wherein the compound of Formula I or a pharmaceutically acceptable salt, or ester thereof is administrated at a daily dose of 100 mg to 1000 mg in term of the compound of Formula I.

8. The compound for use according to any one of claims 1 to 7, wherein the compound of Formula I or a pharmaceutically acceptable salt, or ester thereof is administrated at a daily dose of 100 mg to 500 mg in term of the compound of Formula I.

9. A pharmaceutical composition for use in treating or preventing Coronaviridae infection comprising a compound of Formula I or a stereoisomer, a tautomer, an N-oxide, a solvate, or a pharmaceutically acceptable salt thereof for use according to any one of claims 1 to 8,

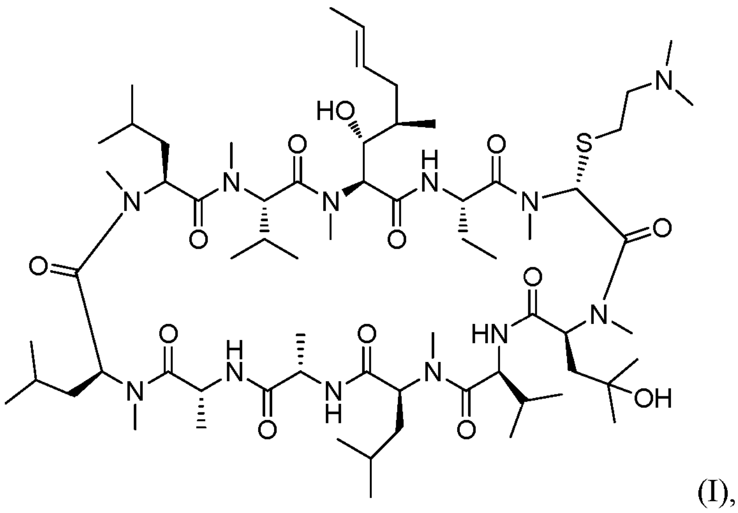

(I),

and the Coronaviridae is 2019-nCov virus.

**10.** The pharmaceutical composition for use according to claim 9, further comprising at least one other therapeutic agent or composition thereof selected from the group comprising a corticosteroid, an anti-inflammatory signal transduction modulator, a β2-adrenoreceptor agonist bronchodilator, an anticholinergic, a mucolytic agent, hypertonic saline, agent for reducing cytokine storm, other drugs for treating a covid-19 virus infection, or mixtures thereof.

**11.** The pharmaceutical composition for use according to claim 9 or 10, wherein pharmaceutical composition is in a form of sol, aromatic water, aerosol, partial inhalant, powder, granule, tablets, ointment, paste, emulsion, suspension, gas dispersion, solid dispersion, micro particle, pill.

**12.** The pharmaceutical composition for use according to any one of claims 9 to 11, further comprising one or more pharmaceutically acceptable carriers, diluents or excipients.

**13.** The pharmaceutical composition for use according to any one of claims 9 to 12, comprising the compound of Formula I or a stereoisomer, a tautomer, an N-oxide, a solvate, or a pharmaceutically acceptable salt thereof at a dose of lower than 1000 mg, 800 mg, 600 mg or 500 mg.

**Patentansprüche**

**1.** Eine Verbindung der Formel I oder ein Stereoisomer, ein Tautomer, ein N-Oxid, ein Solvat oder ein pharmazeutisch akzeptables Salz davon zur Verwendung bei der Behandlung oder Vorbeugung einer Coronaviridae-Infektion bei einem Patienten, wobei dem Patienten eine therapeutisch wirksame Menge der Verbindung der Formel I oder eines Stereoisomers, eines Tautomers, eines N-Oxids, eines Solvats oder eines pharmazeutisch akzeptablen Salzes davon verabreicht wird,

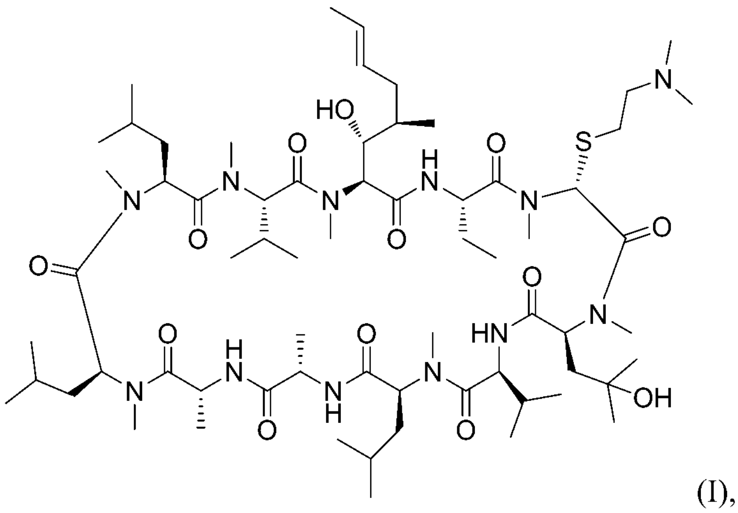

(I),

und das Coronavirus das 2019-nCov-Virus ist.

2. Die Verbindung zur Verwendung gemäß Anspruch 1, wobei eine therapeutisch wirksame Menge mindestens eines weiteren therapeutischen Wirkstoffs oder einer Zusammensetzung davon, ausgewählt aus der Gruppe bestehend aus einem Kortikosteroid, einem entzündungshemmenden Signaltransduktionsmodulator, einem β2-Adrenorezeptoragonisten-Bronchodilatator, einem Anticholinergikum, einem Mukolytikum, einer hypertonischen Kochsalzlösung, ein Mittel zur Verringerung des Zytokinsturms, andere Arzneimittel zur Behandlung einer Covid-19-Virusinfektion oder Mischungen davon ebenfalls an den Patienten verabreicht wird.

3. Die Verbindung zur Verwendung gemäß Anspruch 1 oder 2, wobei die Verbindung der Formel I oder ein Stereoisomer, ein Tautomer, ein N-Oxid, ein Solvat oder ein pharmazeutisch akzeptables Salz davon dem Patienten in Form eines Sols, aromatischen Wassers, Aerosols, Teilinhalationsmittels, Pulvers, Granulats, Tabletten, Salben, Pasten, Emulsionen, Suspensionen, Gasdispersionen, festen Dispersionen, Mikropartikeln oder Pillen verabreicht wird.

4. Die Verbindung zur Verwendung gemäß einem der Ansprüche 1 bis 3, wobei das pharmazeutisch verträgliche Salz mindestens eines ist, ausgewählt aus einer Gruppe, umfassend Phosphat, Hydrochlorid, Hydrobromid, Hydroiodid, Sulfat, Nitrat, Ethansulfonat, Toluolsulfonat und Benzolsulfonat, Acetat, Trifluoracetat, Tartrat, Maleat, Succinat, Citrat, Benzoat, Salicylat und Ascorbat, Adipat, Alginat, Arginat, Aspartat, Bisulfat, Bisulfit, Bromid, Butyrat, Camphorat, Camphersulfonat, Caprylat, Chlorid, Chlorbenzoat, Cyclopentanpropionat, Digluconat, Dihydrogenphosphat, Dinitrobenzoat, Dodecylsulfat, Fumarat, Galacterat, Galacturonat, Glucoheptaoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pektinat, Persulfat, Phenylacetat, 3-Phenylpropionat, Phosphat, Phosphonat und Phthalat.

5. Die Verbindung zur Verwendung gemäß einem der Ansprüche 1 bis 4, wobei die Verbindung der Formel I oder ein Stereoisomer, ein Tautomer, ein N-Oxid, ein Solvat oder ein pharmazeutisch akzeptables Salz davon dem Patienten zusammen mit einem oder mehreren pharmazeutisch akzeptablen Trägern, Verdünnungsmitteln oder Hilfsstoffen verabreicht wird.

6. Die Verbindung zur Verwendung gemäß einem der Ansprüche 1 bis 5, wobei die Verbindung der Formel I oder ein pharmazeutisch akzeptables Salz oder Ester davon in einer Tagesdosis von weniger als 1000 mg, bezogen auf die Verbindung der Formel I, verabreicht wird.

7. Die Verbindung zur Verwendung gemäß einem der Ansprüche 1 bis 6, wobei die Verbindung der Formel I oder ein pharmazeutisch akzeptables Salz oder Ester davon in einer Tagesdosis von 100 mg bis 1000 mg, bezogen auf die Verbindung der Formel I, verabreicht wird.

8. Die Verbindung zur Verwendung gemäß einem der Ansprüche 1 bis 7, wobei die Verbindung der Formel I oder ein pharmazeutisch akzeptables Salz oder Ester davon in einer Tagesdosis von 100 mg bis 500 mg, bezogen auf die

Verbindung der Formel I, verabreicht wird.

**9.** Eine pharmazeutische Zusammensetzung zur Verwendung bei der Behandlung oder Vorbeugung einer Coronaviridae-Infektion, umfassend eine Verbindung der Formel I oder ein Stereoisomer, ein Tautomer, ein N-Oxid, ein Solvat oder ein pharmazeutisch akzeptables Salz davon zur Verwendung nach einem der Ansprüche 1 bis 8,

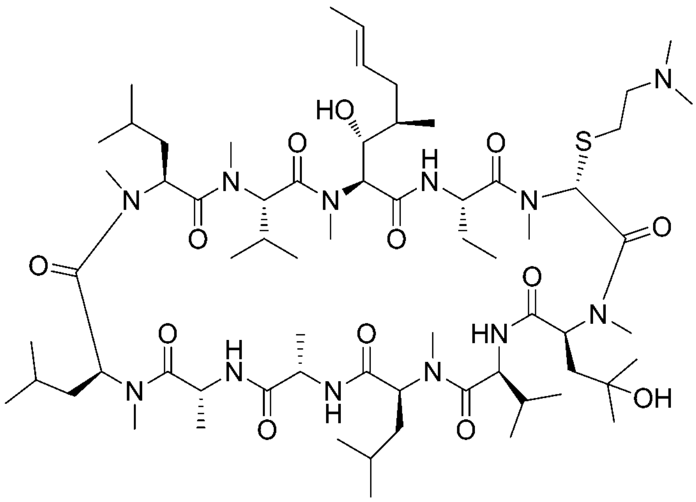

(I),

und das Coronavirus das 2019-nCov-Virus ist.

**10.** Die pharmazeutische Zusammensetzung zur Verwendung gemäß Anspruch 9, die ferner mindestens ein weiteres therapeutisches Mittel oder eine weitere therapeutische Zusammensetzung umfasst, ausgewählt aus der Gruppe, bestehend aus einem Kortikosteroid, einem entzündungshemmenden Signaltransduktionsmodulator, einem β2-Adrenorezeptoragonisten-Bronchodilatator, einem Anticholinergikum, einem mukolytischen Mittel, hypertonischer Kochsalzlösung, einem Mittel zur Verringerung des Zytokinsturms, anderen Arzneimitteln zur Behandlung einer Covid-19-Virusinfektion oder Mischungen davon.

**11.** Die pharmazeutische Zusammensetzung zur Verwendung gemäß Anspruch 9 oder 10, wobei die pharmazeutische Zusammensetzung in Form eines Sols, aromatischen Wassers, Aerosols, Teilinhalationsmittels, Pulvers, Granulats, Tabletten, Salben, Pasten, Emulsionen, Suspensionen, Gasdispersionen, festen Dispersionen, Mikropartikeln oder Pillen vorliegt.

**12.** Die pharmazeutische Zusammensetzung zur Verwendung gemäß einem der Ansprüche 9 bis 11, die ferner einen oder mehrere pharmazeutisch verträgliche Träger, Verdünnungsmittel oder Hilfsstoffe umfasst.

**13.** Die pharmazeutische Zusammensetzung zur Verwendung gemäß einem der Ansprüche 9 bis 12, umfassend die Verbindung der Formel I oder ein Stereoisomer, ein Tautomer, ein N-Oxid, ein Solvat oder ein pharmazeutisch akzeptables Salz davon in einer Dosis von weniger als 1000 mg, 800 mg, 600 mg oder 500 mg.

## Revendications

**1.** Composé de formule I ou stéréoisomère, tautomère, N-oxyde, solvate ou sel pharmaceutiquement acceptable de celui-ci pour une utilisation dans le traitement ou la prévention d'une infection par Coronaviridae chez un sujet, dans lequel une quantité thérapeutiquement efficace du composé de formule I ou d'un stéréoisomère, d'un tautomère, d'un N-oxyde, d'un solvate ou d'un sel pharmaceutiquement acceptable de celui-ci est administrée au sujet,

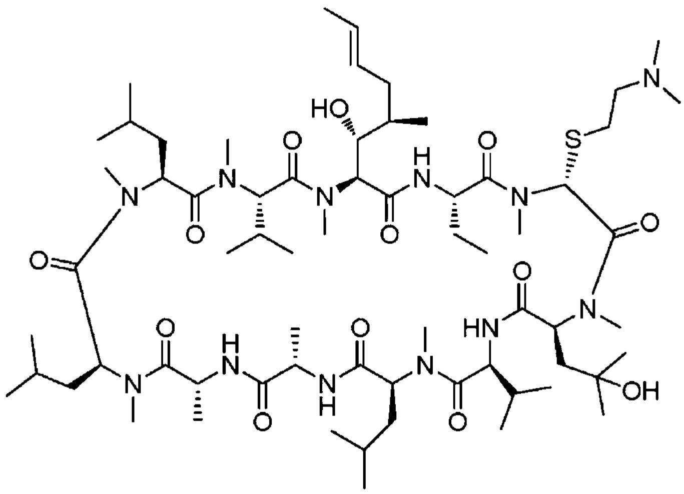

(I),

et le Coronaviridae est le virus 2019-nCov.

**2.** Composé pour une utilisation selon la revendication 1, dans lequel une quantité thérapeutiquement efficace d'au moins un autre agent thérapeutique ou d'une composition de celui-ci sélectionné parmi le groupe comprenant un corticostéroïde, un modulateur de transduction des signaux anti-inflammatoires, un bronchodilatateur agoniste des récepteurs β2-adrénergiques, un anticholinergique, un agent mucolytique, une solution saline hypertonique, un agent de réduction de la tempête cytokinique, d'autres médicaments pour traiter une infection par le virus de la Covid-19, ou des mélanges de ceux-ci, est également administrée au sujet.

**3.** Composé pour une utilisation selon la revendication 1 ou 2, dans lequel le composé de formule I ou un stéréoisomère, un tautomère, un oxyde N, un solvate ou un sel pharmaceutiquement acceptable de celui-ci est administré au sujet sous forme de sol, d'eau aromatique, d'aérosol, d'inhalant partiel, de poudre, de granulés, de comprimés, de pommade, de pâte, d'émulsion, de suspension, de dispersion gazeuse, de dispersion solide, de microparticules, de pilules.

**4.** Composé pour une utilisation selon l'une des revendications 1 à 3, dans lequel le sel pharmaceutiquement acceptable est au moins un élément sélectionné parmi un groupe comprenant un phosphate, un chlorhydrate, un bromhydrate, un iodhydrate, un sulfate, un nitrate, un éthanesulfonate, un toluènesulfonate et un benzènesulfonate, un acétate, un trifluoroacétate, un tartrate, un maléate, un succinate, un citrate, un benzoate, un salicylate et un ascorbate, un adipate, un alginate, un arginate, un aspartate, un bisulfate, un bisulfite, un bromure, un butyrate, un camphorate, un camphorsulfonate, un caprylate, un chlorure, un chlorobenzoate, un cyclopentanepropionate, un digluconate, un dihydrogénophosphate, un dinitrobenzoate, un dodécylsulfate, un fumarate, un galactérate, un galacturonate, un glucoheptaoate, un gluconate, un glutamate, un glycérophosphate, un hémisuccinate, un hémisulfate, un heptanoate, un hexanoate, un hippurate, un chlorhydrate, un bromhydrate, un iodhydrate, un 2-hydroxyéthanesulfonate, un iodure, un iséthionate, un iso-butyrate, un lactate, un lactobionate, un malate, un malonate, un mandélate, un métaphosphate, un méthanesulfonate, un méthylbenzoate, un monohydrogénophosphate, un 2-naphtalènesulfonate, un nicotinate, un nitrate, un oxalate, un oléate, un pamoate, un pectinate, un persulfate, un phénylacétate, un 3-phénylpropionate, un phosphate, un phosphonate et un phtalate.

**5.** Composé pour une utilisation selon l'une des revendications 1 à 4, dans lequel le composé de formule I ou un stéréoisomère, un tautomère, un N-oxyde, un solvate ou un sel pharmaceutiquement acceptable de celui-ci est administré au sujet conjointement avec un ou plusieurs supports, diluants ou excipients pharmaceutiquement acceptables.

**6.** Composé pour une utilisation selon l'une des revendications 1 à 5, dans lequel le composé de formule I ou un sel pharmaceutiquement acceptable, ou un ester de celui-ci, est administré à une dose quotidienne inférieure à 1000 mg en termes de composé de formule I.

**7.** Composé pour une utilisation selon l'une des revendications 1 à 6, dans lequel le composé de formule I ou un sel pharmaceutiquement acceptable, ou un ester de celui-ci, est administré à une dose quotidienne de 100 mg à 1000 mg

en termes de composé de formule I.

**8.** Composé pour une utilisation selon l'une des revendications 1 à 7, dans lequel le composé de formule I ou un sel pharmaceutiquement acceptable, ou un ester de celui-ci, est administré à une dose quotidienne de 100 mg à 500 mg en termes de composé de formule I.

**9.** Composition pharmaceutique pour une utilisation dans le traitement ou la prévention d'une infection par Coronaviridae comprenant un composé de formule I ou un stéréoisomère, un tautomère, un N-oxyde, un solvate, ou un sel pharmaceutiquement acceptable de celui-ci pour une utilisation selon l'une des revendications 1 à 8,

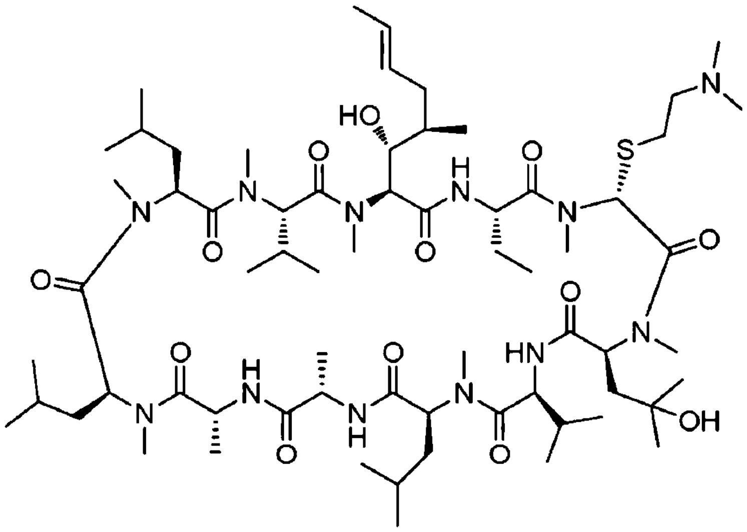

(I),

et le Coronaviridae est le virus 2019-nCov.

**10.** Composition pharmaceutique pour une utilisation selon la revendication 9, comprenant en outre au moins un autre agent thérapeutique ou une composition de celui-ci sélectionné parmi le groupe comprenant un corticostéroïde, un modulateur de transduction des signaux anti-inflammatoires, un bronchodilatateur agoniste des récepteurs β2-adrénergiques, un anticholinergique, un agent mucolytique, une solution saline hypertonique, un agent de réduction de la tempête cytokinique, d'autres médicaments pour traiter une infection par le virus de la Covid-19, ou des mélanges de ceux-ci.

**11.** Composition pharmaceutique pour une utilisation selon la revendication 9 ou 10, dans laquelle la composition pharmaceutique est sous forme de sol, d'eau aromatique, d'aérosol, d'inhalant partiel, de poudre, de granulés, de comprimés, de pommade, de pâte, d'émulsion, de suspension, de dispersion gazeuse, de dispersion solide, de microparticules, de pilules.

**12.** Composition pharmaceutique pour une utilisation selon l'une des revendications 9 à 11, comprenant en outre un ou plusieurs supports, diluants ou excipients pharmaceutiquement acceptables.

**13.** Composition pharmaceutique pour une utilisation selon l'une des revendications 9 à 12, comprenant le composé de formule I ou un stéréoisomère, un tautomère, un N-oxyde, un solvate, ou un sel pharmaceutiquement acceptable de celui-ci à une dose inférieure à 1000 mg, 800 mg, 600 mg ou 500 mg.

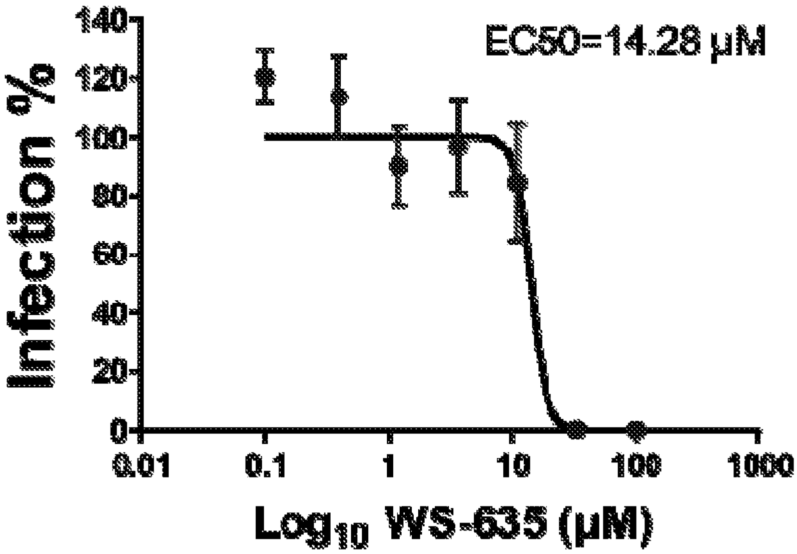


Figure 1

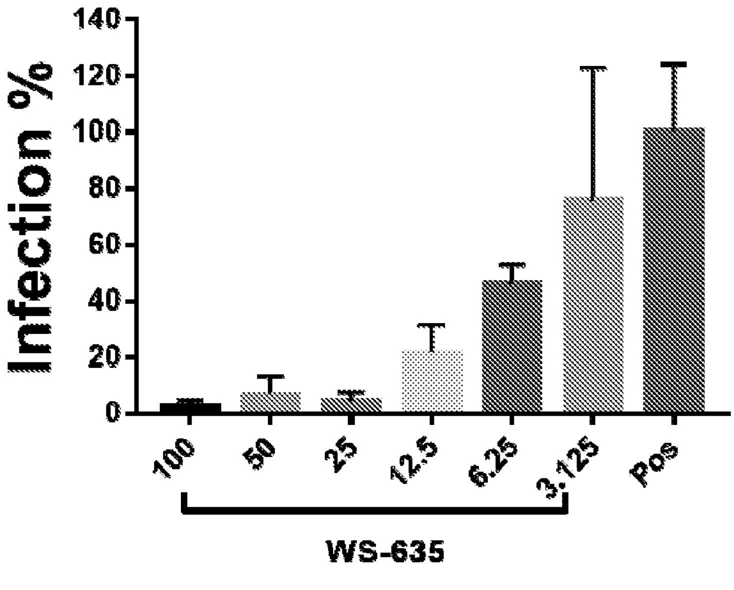


Figure 2

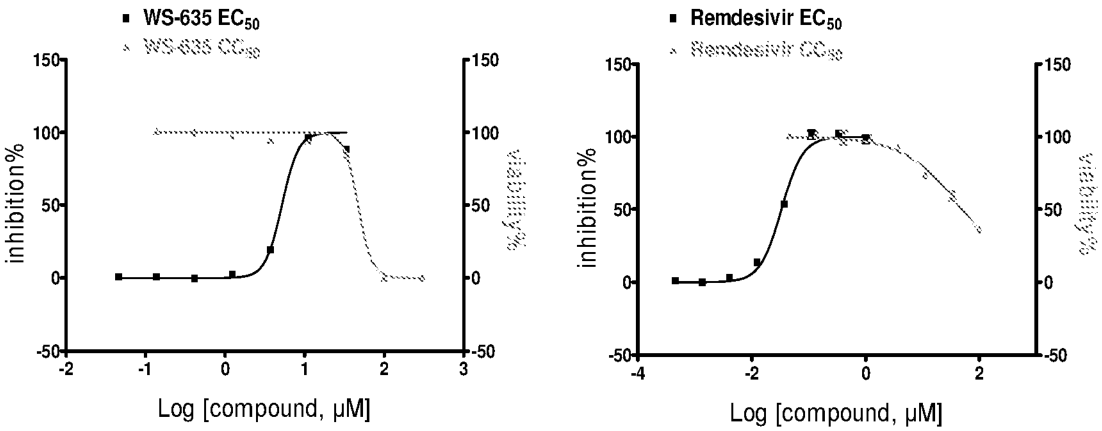
WS-635 EC50
WS-635 CC50
inhibition%
viability%
Log [compound, μM]
Remdesivir EC50
Remdesivir CC50
inhibition%
viability%
Log [compound, μM]

Figure 3